# EUROPEAN PATENT APPLICATION

(11) **EP 1 801 045 A1**
(43) Date of publication of application: **27.06.2007**
(21) Application number: 05785967.0
(22) Date of filing: 21.09.2005
(51) Int. Cl.: B65G 47/86, B65G 17/46, B65G 47/52

(54) **CONVEYANCE DEVICE**

(30) Priority: 22.09.2004 JP 2004274980
(71) Applicant: Hallys Corporation, Akashi-shi Hyogo 6740064 (JP)
(72) Inventor: Aoyama, Hiroshi, Hallys Corporation, Akashi-shi, Hyogo 6740064 (JP); Nishigawa, Ryoichi, Hallys Corporation, Akashi-shi, Hyogo 6740064 (JP)
(74) Representative: Kramer - Barske - Schmidtchen
(86) International application number: PCT/JP2005/017421
(87) International publication number: WO 2006/033369

(57) **Abstract**

A conveying apparatus 1 comprises: a first conveying device 3 and a second conveying device 5 for holding and conveying a work 2; and transfer device 6 configured to receive the work 2 from the first conveying device 3 and transfer the received work 2 to the second conveying device 5. The transfer device 6 has two or more end-effectors 71 to 76 that revolve along the same circumference to convey the work 2, and each of the end-effectors 71 to 76 is configured to revolve independently of at least any of the other end-effectors.

## Description

### Technical Field

The present invention relates to a conveying apparatus that conveys works.

### Background Art

There has been known a conveying apparatus having a function of, for example, adjusting conveying positions of works (for example, refer to Patent Document 1). An example of the conveying apparatus includes, on a conveying surface, a suction mechanism that sucks a work and a protrusion that locks the work. In the conveying apparatus, a suction force of the work suction mechanism is reduced in an inclined zone provided in a conveying path, and the work is slid downward of an inclined surface by the effect of gravity to be locked by the protrusion, thereby adjusting a conveying position of the work.

The conventional conveying apparatus, however, has a problem that a level difference needs to be provided in the conveying path, which may increase the size of the device. Further, the conveying position of the work is uniquely determined according to a position of the protrusion provided on the conveying surface, which may prevent high flexibility of adjustment of the conveying position from being maintained.

Patent Document 1: Japanese Patent Laid-Open No. 2001-335135

The present invention is achieved in view of the problem of the conventional conveying apparatus, and has an object to provide a conveying apparatus that can adjust a conveying position of a work with high flexibility and accuracy.

### Disclosure of the Invention

The present invention provides a conveying apparatus comprising: first and second conveying devices configured to hold and convey a work; and a transfer device configured to receive the work from the first conveying device and transfer the work to the second conveying device,
wherein the transfer device comprises two or more end-effectors that revolve along the same circumference to convey the work, and each of the end-effectors is configured to revolve independently of at least any of the other end-effectors.

In the conveying apparatus of the present invention, the each end-effector can revolve independently of at least any of the other end-effectors. Thus, the each end-effector can receive the work in response to changes in conveying speed or conveying position, or the like of the work conveyed by the first conveying device. Further, the each end-effector can transfer the work with high accuracy in response to a conveying speed or a target conveying position, or the like conveyed by the second conveying device.

Specifically, the conveying apparatus of the present invention allows the work to be received and transferred with high flexibility as compared with the case where all the end-effectors integrally revolve. This accommodates variations in conveying position of the work in the first conveying device and allows the work to be conveyed in the second conveying device with high positional accuracy. Particularly, when all the end-effectors are configured to be able to revolve independently of one another, the operation and effect of the present invention can be further improved.

In the conveying apparatus, speed control of the end-effector during revolution allows revolution position control of the each end-effector. Thus, the conveying apparatus allows adjustment of timing for the each end-effector to receive the work. This can accommodate variations in conveying position of the work conveyed by the first conveying device. Also, revolution position control of the end-effector during revolution allows adjustment of timing for the each end-effector to transfer the work to the second conveying device. This allows the conveying position of the work in the second conveying device to be adjusted with high accuracy.

As described above, with the conveying apparatus of the present invention, the operation of the transfer device provided between the first conveying device and the second conveying device can accommodate variations in conveying position in the first conveying device, and allows the work to be transferred to the second conveying device with high positional accuracy.

### Brief Description of the Drawings

Figure 1 is a block diagram of a conveying apparatus in Embodiment 1;
Figure 2 is a front view of a transfer device in Embodiment 1;
Figure 3 is a sectional view of a sectional structure of the transfer device in Embodiment 1, viewed along arrows A-A in Figure 2;
Figure 4 is a perspective view of an assembling structure of a coaxial rotor in Embodiment 1;
Figure 5A is a sectional view showing one first coaxial rotor individually in Embodiment 1;
Figure 5B is a sectional view showing one second coaxial rotor individually in Embodiment 1;
Figure 5C is a sectional view showing one third coaxial rotor individually in Embodiment 1;
Figure 6 illustrates end-effectors arranged on the same circumference in Embodiment 1;
Figure 7 is a graph illustrating revolution of the end-effectors in Embodiment 1;
Figure 8 is a graph illustrating revolution of one end-effector in Embodiment 1; and
Figure 9 is a block diagram of a conveying apparatus in Embodiment 2.

### Description of Symbols

- 1: conveying apparatus
- 10: coaxial rotor
- 2: work
- 3: first conveying device
- 5: second conveying device
- 6, 6a, 6b: transfer device
- 70: suction pad
- 71 to 76: end-effector

### Best Mode for Carrying Out the Invention

The conveying apparatus of the present invention has the plurality of end-effectors that revolve along the same circumference. With the plurality of end-effectors, the works can be conveyed with extremely high efficiency. For example, with six end-effectors controlled each revolution independently, the work can be conveyed at a high speed cycle of one sixth of a revolution cycle of the each end-effector.

The conveying apparatus can be used for conveying, for example, adhesive tapes that are components of disposable diapers, sanitary napkins, or the like, or electronic components such as interposers that are components of RF-TAGs, or the like. Further, a counterpart member on which the adhesive tapes or the interposers as the works are placed may be conveyed by the second conveying device. In this case, the works can be transferred to the counterpart member under conveyance by the conveying apparatus, thereby increasing production efficiency.

It is preferable that the transfer device includes coaxial rotors that integrally hold one or more end-effectors revolving therewith, and three or more bearings placed coaxially so as to rotatably support at least two coaxial rotors, each of the bearings includes a substantially cylindrical inner ring, a substantially cylindrical outer ring fitted from outside to the inner ring, and a bearing mechanism that allows relative rotation between the inner ring and the outer ring,
the inner ring of one or more middle bearings placed in an axially middle position among the bearings is connected to the outer ring of adjacent another bearing and is configured to integrally rotate therewith, and integrally holds any of the coaxial rotors,
the inner ring of one of bearings placed at axial ends among the bearings is connected to the outer ring of adjacent another bearing and is configured to integrally rotate therewith, and integrally holds any of the coaxial rotors, and the outer ring thereof is secured to a structure member of the transfer device,
the outer ring of the other of the bearings placed at the axial ends among the bearings is connected to the inner ring of adjacent another bearing and is configured to integrally rotate therewith, and the inner ring thereof is secured to a structure member of the transfer device, and
the outer rings integrally connected to the inner rings of the adjacent bearings among the outer rings are connected to an output shaft of an external motor whose rotation is independently controlled.

In this case, the inner ring of one of the adjacent bearings and the outer ring of the other of the bearings are connected to achieve an integral bearing structure including the plurality of bearings. Specifically, a structure can be achieved in which the each coaxial rotor supports the other coaxial rotors.
Further, with such a support structure, a rotation driving force supplied via the outer ring can rotatably drive the coaxial rotor integrally held by the inner ring connected to the outer ring. In case three coaxial rotors are included, application of the rotation driving force from three directions allows an external pressure (stress) toward an axis acting on each bearing to be averaged and reduced.
As the external motor, a servo-controlled motor may be used, and a direct drive mechanism that can achieve control with high accuracy may be used. The output shaft of the external motor and the outer ring can be directly connected, or indirectly connected via a gear or a timing belt.

It is preferable that the conveying apparatus comprises control means for controlling a revolution speed and a revolution position in revolution of the each end-effector,
the control means is configured to perform control so that a revolution speed of the end-effector when receiving the work from the first conveying device substantially matches a conveying speed of the first conveying device, and a revolution speed of the end-effector when transferring the work to the second conveying device substantially matches a conveying speed of the second conveying device.

In this case, the end-effectors that revolve along the same circumference in the conveying apparatus synchronize with a conveying motion of the first conveying device while maintaining the order of revolution thereof, and can receive the work from the first conveying device at a relative speed of substantially zero. Then, the end-effectors synchronize with a conveying motion of the second conveying device, and can transfer the work to the second conveying device at a relative speed of substantially zero.

The transfer device in the conveying apparatus can continuously receive the works continuously conveyed, and then continuously transfer the received works to the second conveying device without stopping revolution of the end-effectors.

In the conveying apparatus, the relative speed between each of the conveying device and the end-effector is substantially zero in receipt or transfer of the work. Thus, the conveying apparatus can transfer the work with extremely high accuracy and extremely low possibility of damaging the work during conveyance.

Particularly, the operation and effect of the present invention is more effective when a difference in conveying speed exists between the first conveying device and the second conveying device. In this case, the end-effectors during revolution are variably controlled in their speed as needed to respond to respective conveying speeds with high accuracy. If the relative speed between the end-effector and the conveying device is substantially zero in receipt and transfer of the work, high conveying position accuracy of the work can be maintained.

The conveying apparatus preferably comprises a first measuring portion configured to detect a conveying position and a conveying speed of the work conveyed by the first conveying device.
In this case, revolution of the end-effector can be controlled based on the conveying position and the conveying speed of the work conveyed by the first conveying device. For example, as compared with the case where the end-effector is controlled using indirect information such as control information of the first conveying device, or the like, the work can be received at higher speed with higher accuracy.

It is preferable that the second conveying device has a conveying surface on which marks are provided, the marks indicating target conveying positions on which the works are transferred by the transfer device, and the transfer device includes a second measuring portion configured to detect the target conveying positions in the second conveying device and a movement speed thereof.
In this case, revolution of the end-effector can be controlled based on the target conveying positions in the second conveying device or the movement speed thereof. For example, as compared with the case where the end-effector is controlled using indirect information such as control information of the second conveying device, or the like, the work can be transferred at higher speed with higher accuracy.

It is preferable that a difference between the conveying speed of the first conveying device and the conveying speed of the second conveying device is 80% to 400% of the conveying speed of the first conveying device.
In this case, the large difference exists between the conveying speed of the first conveying device and the conveying speed of the second conveying device, and thus the operation and effect of the present invention is particularly effective.

It is preferable that the first and the second conveying device each include a roller or a translating conveyor belt, and are configured to convey the work placed on the roller or the conveyer belt.
In this case, the conveying apparatus with high conveying efficiency can be configured using the roller or the conveyor belt.

### (Embodiment 1)

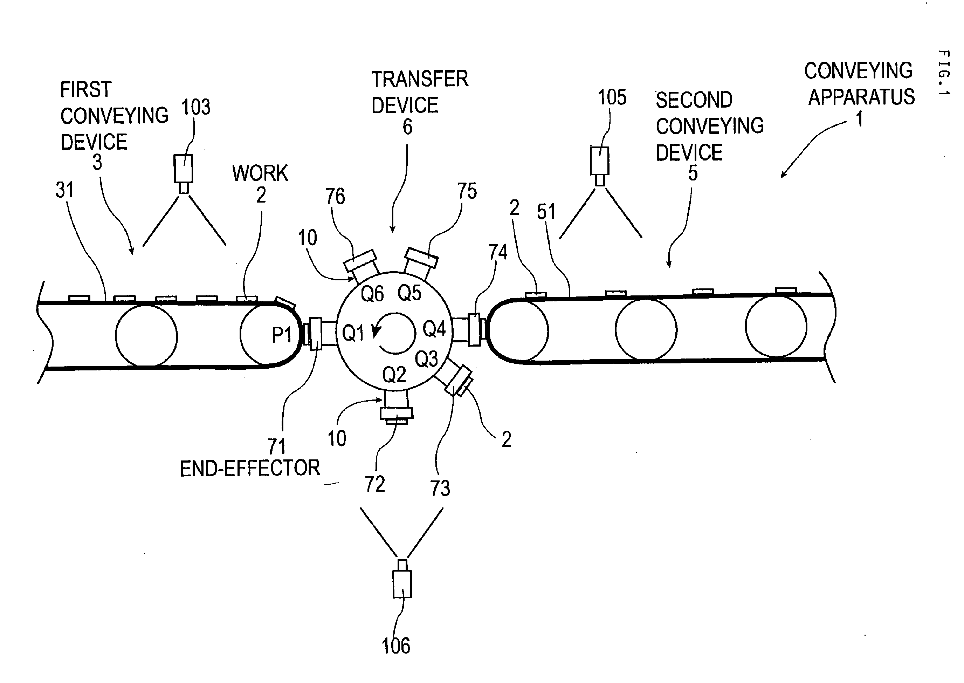

This embodiment relates to a conveying apparatus 1 having a function of adjusting a conveying position of a work 2. This will be described with reference to Figures 1 to 8.
As shown in Figure 1, the conveying apparatus 1 in the embodiment includes first and second conveying device 3 and 5 for holding and conveying the work 2, and transfer device 6 configured to receive the work 2 from the first conveying device 3 and transfer the work 2 to the second conveying device 5.
The transfer device 6 has two or more end-effectors 71 to 76 that revolve along the same circumference to convey the work 2, and each of the end-effectors 71 to 76 is configured to revolve independently of at least any of the other end-effectors 71 to 76. In the embodiment, all the end-effectors 71 to 76 are configured to revolve independently of one another.
Now, this will be described in more detail.

As shown in Figure 1, the first conveying device 3 and the second conveying device 5 have conveyor belts 31 and 51, respectively. The conveying devices 3 and 5 are configured to hold the works 2 as water-absorbing pads on surfaces of the conveyor belts 31 and 51, and convey the works. The transfer device 6 is constituted by a combination of six coaxial rotors 10 integrally holding the respective end-effectors 71 to 76. The end-effectors 71 to 76 are configured to transfer the works 2 from the first conveying device 3 to the second conveying device 5.
The conveying apparatus 1 in the embodiment may be configured to convey tapes, nonwoven fabric, cases, food products, IC chips, or the like as works instead of the works 2 as the pads in the embodiment.

The first and the second conveying device 3 and 5 are controlled so as to operate at substantially constant speed by a drive source and a drive control system that are not shown. In the embodiment, a conveying speed of the second conveying device 5 is set to twice a conveying speed of the first conveying device 3.

As shown in Figure 1, the first conveying device 3 is configured to transfer the work 2 to the end-effector (the end-effector 71 in Figure 1) at a point P1. The first conveying device 3 holds the works 2 on the surface of the conveyor belt 31 at substantially regular intervals. A hole (not shown in figures) communicating with a port of an unshown pump is provided in the surface of the conveyor belt 31. The first conveying device 3 sucks the work 2 with the hole in the surface of the conveyor belt 31 under negative pressure. On the other hand, the work 2 is transferred at the point P1 with the hole in the surface of the conveyor belt 31 under positive pressure or atmospheric pressure. The first conveying device 3 may be configured to have a function of cutting and separating a continuous material (a continuous sheet material) into individual pieces of works 2.

As shown in Figure 1, the second conveying device 5 has the conveyor belt 51. The second conveying device 5 holds the works 2 on the surface of the conveyor belt 51 at substantially regular intervals. A hole (not shown in figures) communicating with a port of an unshown pump is provided in the surface of the conveyor belt 51 as in the first conveying device 3. The second conveying device 5 sucks the work 2 with the hole in the surface of the conveyor belt 51 under negative pressure.

The conveying apparatus 1 includes an imaging device (a measuring portion) 103 for photographing a conveying state of the work 2 under conveyance by the first conveying device 3 and obtaining image data. In the embodiment, the image data is subjected to image processing to detect a conveying position and a conveying speed of the work 2 under conveyance. Revolution of the end-effectors 71 to 76 is controlled based on the detected conveying position and conveying speed.

Further, as shown in Figure 1, the conveying apparatus 1 in the embodiment includes an imaging device (a measuring portion) 106 for photographing a state of the works 2 held by the end-effectors 71 to 76, and an imaging device (a measuring portion) 105 for photographing a conveying state of the works 2 in the second conveying device 5. Based on image data obtained by photographing with the imaging devices 105 and 106, for example, abnormalities of the work can be detected such as an abnormal conveying interval, an abnormal attitude, or foreign matter.
Optical sensors may be used instead of the imaging devices 103, 105 and 106. In this case, the conveying apparatus 1 may be configured at low costs using inexpensive optical sensors as compared with imaging devices.

It is preferable to apply marks for image recognition that indicates the target conveying position on the surface of the conveyor belt 51, that is, a conveying surface. In this case, the revolution of the end-effectors 71 to 76 is controlled based on the target conveying positions indicated by the marks, thereby allowing the works 2 to be transferred from the transfer device 6 to the second conveying device 5 with high positional accuracy.

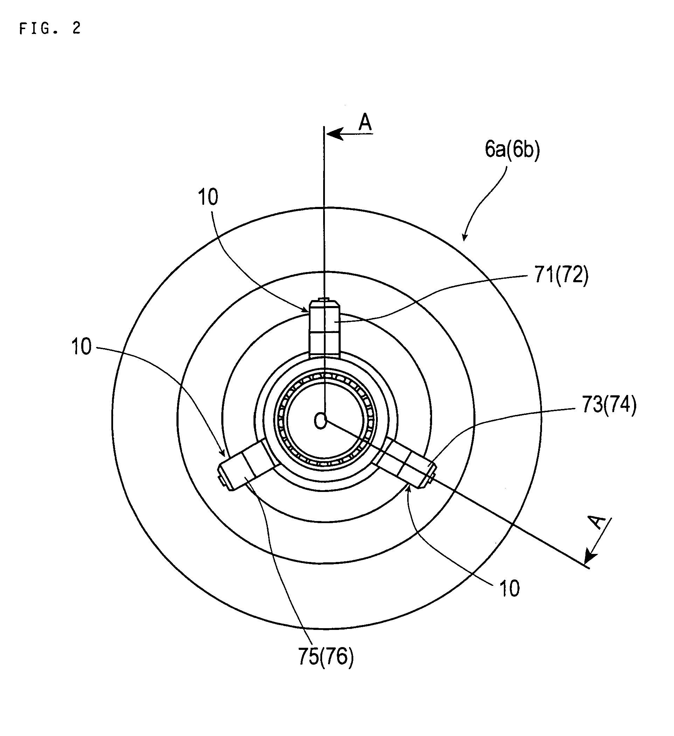

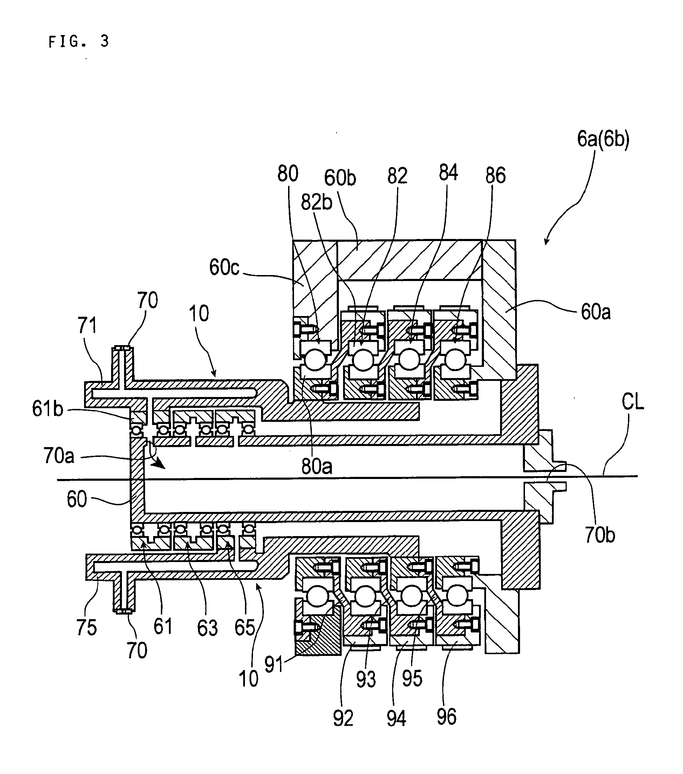

The transfer device 6 is constituted by a combination of two transfer devices 6a and 6b having the same specifications. As shown in Figures 2 to 5, the transfer device 6a and 6b each are constituted by a combination of three coaxial rotors 10. As shown in Figures 2 and 3, the transfer device 6a (6b) includes structure members 60a, 60b and 60c, and four bearings 80, 82, 84 and 86 supported by the structure members 60a, 60b and 60c and coaxially placed. A hollow shaft 60 as a structure member is provided on an inner peripheral side of the bearings 80, 82, 84 and 86. On an outer peripheral side of the hollow shaft 60, bearings 61, 63 and 65 for supporting revolution of the coaxial rotors 10 are placed.

The transfer devices 6a and 6b each have a structure in which the coaxial rotors 10 support one another. Specifically, an inner ring of one of axially adjacent bearings and an outer ring of the other bearing are integrally connected via connecting members. Among the combination of the inner ring and the outer ring connected via the connecting member 91, 93 or 95, the coaxial rotor 10 is integrally secured to an inner peripheral side of the inner ring, and drive wheels 92, 94 or 96 is fitted over the outer ring and secured.

In the transfer device 6a (6b) in the embodiment thus configured, a rotation driving force is supplied to the three drive wheels 92, 94 and 96 from three directions at circumferentially equally spaced intervals around the revolution axis CL. This cancels external pressure of the drive wheels 92, 94 and 96 of the coaxial rotors 10 toward the axis. In the transfer device 6a (6b), general purpose servo-controlled motors (external motors) are connected independently to the drive wheels 92, 94 and 96. This allows independent control of revolution of the coaxial rotors 10 (the end-effectors 71, 73 and 75 (72, 74 and 76)).

In the transfer device 6a (6b) of the embodiment, open space is provided on the outer peripheral side of the drive wheels 92, 94 and 96 in structure thereof. Thus, various mechanisms can be provided on the outer peripheral side of the drive wheels 92, 94 and 96. For example, a direct drive mechanism that allows more accurate control may be provided instead of the general purpose servo-controlled motor. In the embodiment, conductive meshing grooves (precision gears or the like) are provided in the outer peripheral surfaces of the drive wheels 92, 94 and 96 so that the drive wheels 92, 94 and 96 can be rotatably driven by a timing belt.

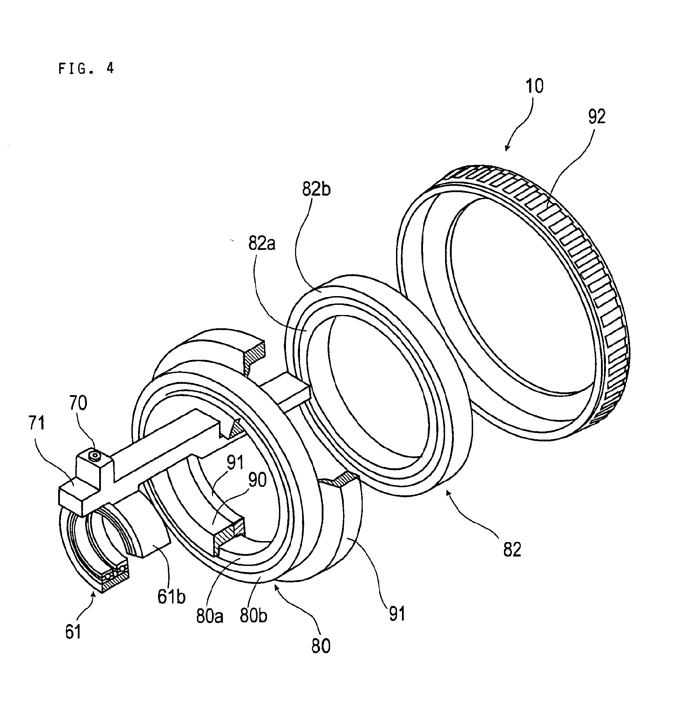

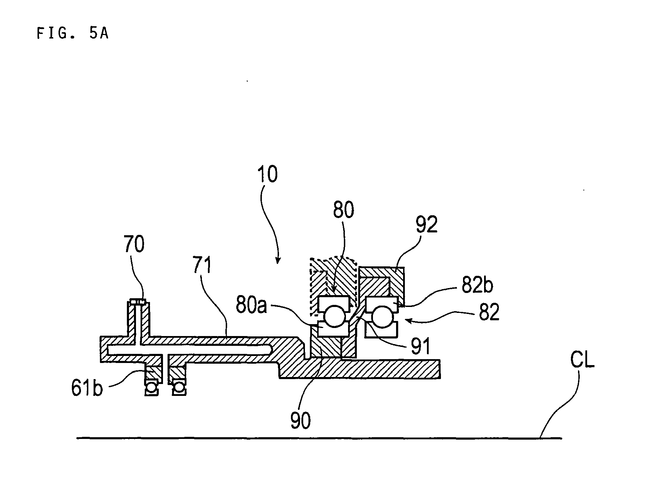

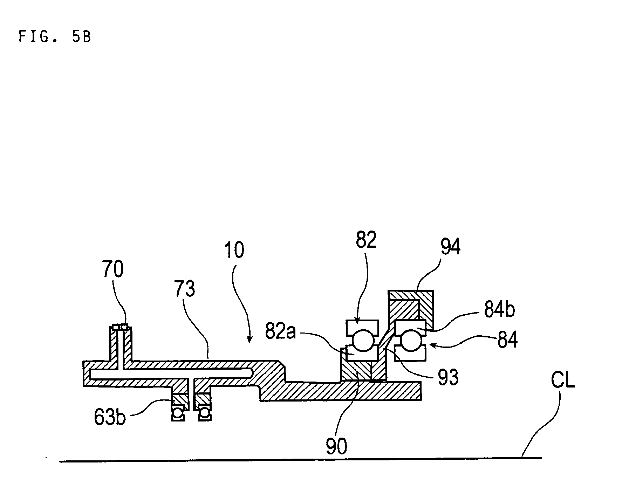

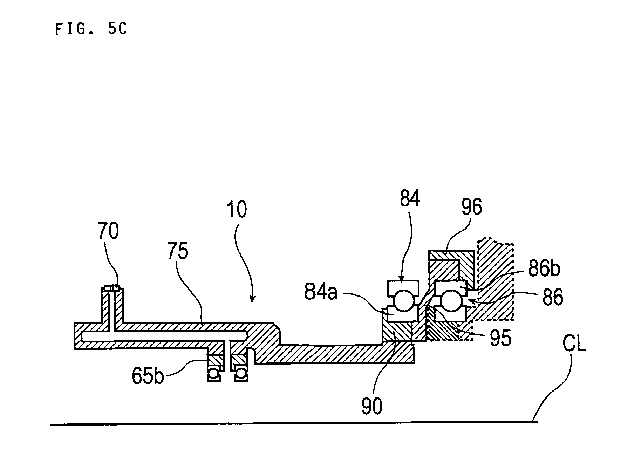

As shown in Figures 4 and 5, each coaxial rotor 10 has one of end-effectors 71 to 76. As shown in Figures 2 and 3, the end-effectors 71, 73 and 75 (72, 74 and 76) are rod members offset placed substantially in parallel with the revolution axis CL. The end-effector 71, 73, 75 (72, 74, 76) are supported revolvably around the revolution axis CL. As shown in Figure 3, the end-effectors 71, 73 and 75 (72, 74 and 76) each have, at a tip thereof, a suction pad 70 for sucking and holding the work 2 (see Figure 1). The suction pad 70 has a hole for controlling air pressure, and is configured to suck and hold the work 2 under negative pressure. On the other hand, when the work 2 is transferred to the second conveying device 5, atmospheric pressure or positive pressure is formed in the hole in the suction pad 70. A structure for achieving control of pressure in the hole in the suction pad 70 will be described later.

As shown in Figures 4 and 5, the end-effector 71 is secured on the tip side thereof (on the side of the suction pad 70) to an outer periphery of an outer ring 61b of the bearing 61, and on the rear end side thereof to an inner periphery of an inner ring 80a of the bearing 80. The inner ring 80a of the bearing 80 is integrally connected to an outer ring 82b of the axially adjacent bearing 82 via the connecting member 91. The drive wheel 92 is secured on the outer peripheral side of the outer ring 82b via part of the connecting member 91.

As shown in Figure 5, the end-effector 73 is secured on the tip side thereof (on the side of the suction pad 70) to an outer periphery of an outer ring 63b of the bearing 63, and on the rear end side thereof to an inner periphery of an inner ring 82a of the bearing 82. The inner ring 82a of the bearing 82 is integrally connected to an outer ring 84b of the axially adjacent bearing 84 via the connecting member 93. The drive wheel 94 is secured on the outer peripheral side of the outer ring 84b via part of the connecting member 93.

As shown in Figure 5, the end-effector 75 is secured on the tip side thereof (on the side of the suction pad 70) to an outer periphery of an outer ring 65b of the bearing 65, and on the rear end side thereof to an inner periphery of an inner ring 84a of the bearing 84. The inner ring 84a of the bearing 84 is integrally connected to an outer ring 86b of the axially adjacent bearing 86 via the connecting member 95. The drive wheel 99 is secured on the outer peripheral side of the outer ring 86b via part of the connecting member 95.

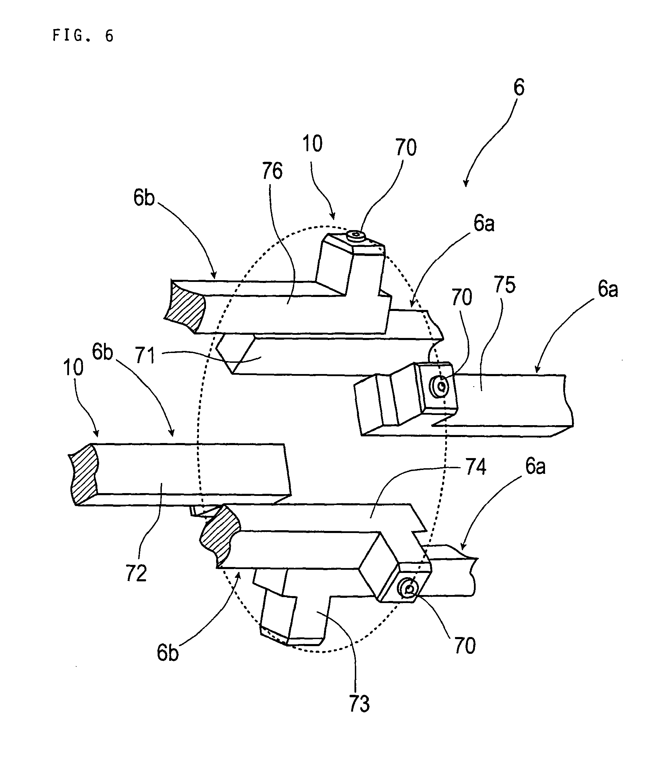

As shown in Figure 6, the conveying apparatus 1 in the embodiment is constituted by the combination of the two transfer devices 6a and 6b. The transfer device 6a includes the end-effectors 71, 73 and 75. The transfer device 6b includes the end-effectors 72, 74 and 76. As shown in the figure, the two transfer devices 6a and 6b are placed to face each other so that all the end-effectors 71 to 76 revolve along the same circumference.

The end-effectors 71 to 76 of the transfer device 6 thus configured synchronize with a conveying motion of the first conveying device 3 while maintaining the sequential order of revolution thereof, and receive the works 2 from the first conveying device 3 at a relative speed of substantially zero. Then, the end-effectors 71 to 76 are configured to synchronize with a conveying motion of the second conveying device 5, and transfer the works 2 to the second conveying device 5 at a relative speed of substantially zero.

The end-effectors 71 to 76 are independently subjected to cycle speed control during revolution including receipt and transfer of the works 2. Specifically, on a revolution path of the end-effectors 71 to 76, timing adjustment (revolution position adjustment) for receipt and transfer of the works 2 and the cycle speed control for adjusting the speed in receipt and transfer are performed.

Next, a suction mechanism (a pressure control mechanism of the hole) of the suction pad 70 in the coaxial rotor 10 will be described with reference to Figure 1. For example, the conveying apparatus 1 in Figure 1 is in the state where the end-effector 71 is receiving the work 2 from the first conveying device 3 (a revolving position Q1), while the end-effectors 72 and 73 are moving toward the second conveying device 5 (revolving positions Q2 and Q3), and the end-effector 74 is transferring the work 2 to the second conveying device 5 (a revolving position Q4). The end-effectors 75 and 76 are rotationally moving (revolving positions Q5 and Q6).

As shown in Figure 3, a through hole 70b is provided along the revolution axis CL in an end surface of the hollow shaft 60 of the transfer device 6a (6b). An intake port of an unshown pump is connected to the through hole 70b. Thus, a hollow portion of the hollow shaft 60 is maintained under negative pressure by the action of the pump. A through hole 70a passing radially is provided in an outer peripheral wall surface of the hollow shaft 60. Holes passing radially and communicating with hollow portions of the end-effectors 71, 73 and 75 are provided in the bearings 61, 63 and 65 so as to communicate with the through hole 70a.

Particularly, the through hole 70a in the embodiment is provided in a predetermined circumferential position so as to communicate with the end-effectors positioned in a revolution zone from the revolving position Q1 (strictly, a position short of the revolving position Q 1 so that the suction pad 70 sucks the work 2 in the revolving position Q1) to the revolving position Q4 (strictly, a position short of the revolving position Q4 so that the suction pad 70 releases the work 2 in the revolving position Q4).

An atmospheric pressure introducing port (not shown in figures) is provided in a predetermined circumferential position in the outer peripheral wall surface of the hollow shaft 60 so as to introduce atmospheric pressure through the hole in the bearing 61 when the end-effector is positioned in the revolving position Q4. Thus, in the transfer device 6a in the embodiment, pressure control of the hole in the suction pad 70 is automatically performed in response to rotation of the bearings 61, 63 and 65 caused by the revolution of the end-effectors 71, 73 and 75.

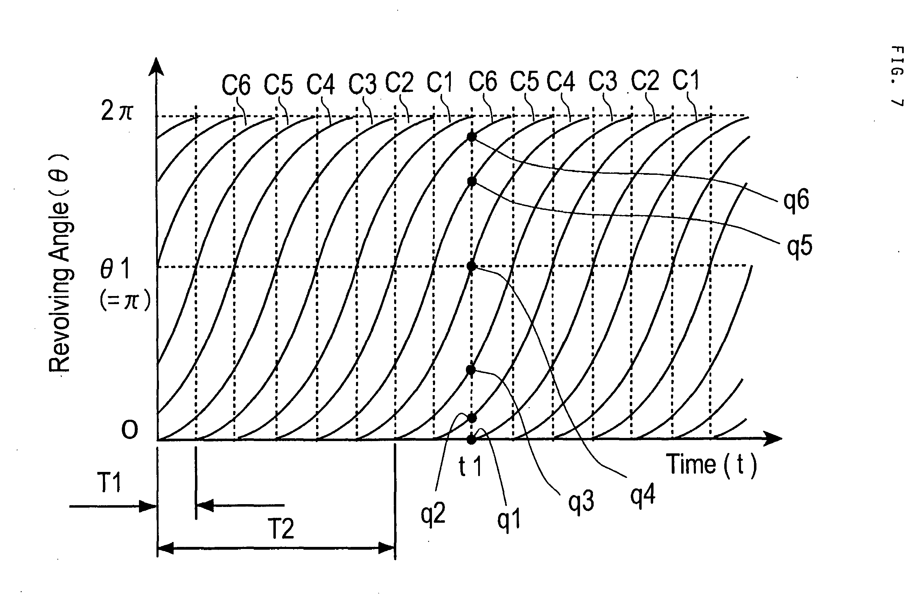

Next, revolution of the end-effectors 71 to 76 will be described. Figure 7 shows changes with time in revolving angles of the end-effectors 71 to 76. Curves Cl to C6 correspond to revolving movements of the end-effectors 71 to 76, respectively in Figure 1. Points q1 to q6 at time t1 on the graph correspond to the revolving positions Q1 to Q6, respectively in Figure 1. A contact position between the first conveying device 3 and the transfer device 6 (the revolving position Q1) in Figure 1 is a home position of a revolving angle q, and a revolving direction is counterclockwise as shown in Figure 1.

A cycle T1 in Figure 7 is a cycle of the first conveying device 3 supplying the work 2 to the transfer device 6 (a work supply cycle). The work supply cycle is determined by the conveying speed of the first conveying device 3, and the interval between the works 2 on the conveyor belt 31. A cycle T2 is a cycle of revolution of each end-effector. In the embodiment, the six end-effectors 71 to 76 revolution-controlled independently are used. Thus, the transfer device 6 in the embodiment can accommodate a work supply speed about six times higher than a revolution speed of each end-effector. Specifically, a relationship of T2 ≈ 6 x T1 is met in a short time period, and an average relationship in a long time period is T2 = 6 x T1.

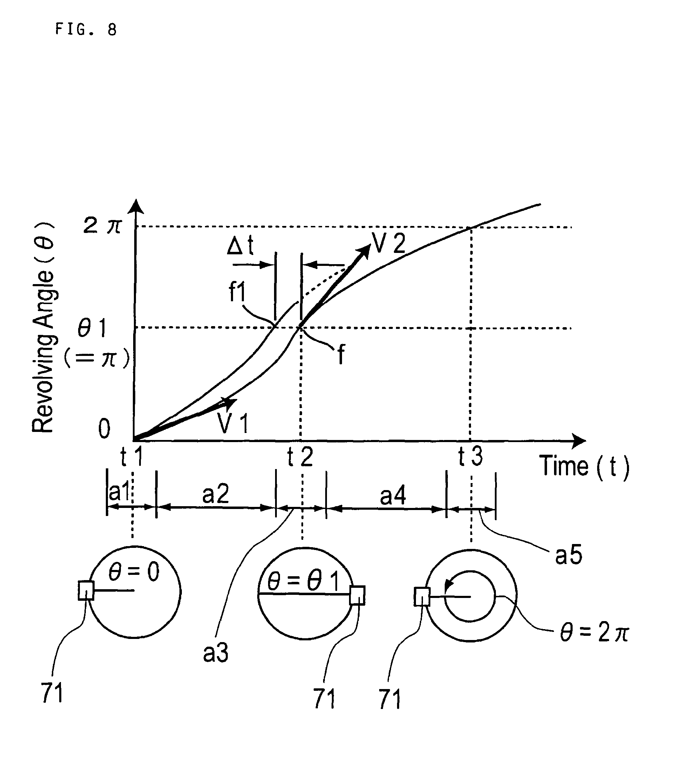

The revolution of the end-effector 71, by way of example, will be described. Figure 8 shows changes with time in the revolving angle of the end-effector 71 during revolution. The end-effector 71 receives the work 2 from the first conveying device 3 at speed V 1 at the time t = tl with the revolving angle q = 0. Then, the end-effector 71 transfers the work 2 to the second conveying device 5 at speed V2 at the time t = t2 with the revolving angle q = q1 (= p). Then, the end-effector 71 returns to an initial revolving position at the time t = t3 (= tl + T2) with the revolving angle of 2p.

Time zones a1, a3 and a5 synchronize with the conveying motion of the first conveying device 3 or the conveying motion of the second conveying device 5 for receipt or transfer of the work 2. In these time zones, the speed is maintained substantially constant so that a relative speed to the conveying speed of the work 2 is substantially zero. On the other hand, in the time zones a2 and a4, the revolving speed of the end-effector 71 is increased or reduced.

In the time zones a2 and a4, the revolution position is adjusted besides the adjustment of the speed. As shown in Figure 8, the revolution position adjustment is performed, for example, when the conveying speed of the second conveying device 5 varies. In order to maintain a constant conveying interval in the second conveying device 5 when the conveying speed of the second conveying device 5 varies, timing for transferring the work 2 from the transfer device 6 to the second conveying device 5 needs to be adjusted. Thus, in order to adjust the timing, control of the revolution positions of the end-effectors 71 to 76 is performed.

For example, if a need arises to transfer the work 2 earlier by time Dt, the speed of the end-effector 71 is increased to cause the curve in Figure 8 to pass a point f1 instead of a point f. This allows the work 2 to be transferred with high accuracy to a predetermined position such that a substantially constant conveying interval of the second conveying device 5 can be maintained.

As described above, the conveying apparatus 1 in the embodiment independently controls the revolution of the end-effectors 71 to 76 to achieve transfer at high speed with high accuracy.
The conveying apparatus in the embodiment can be used in various converting machines, printers, labelers, semiconductor producing devices, or the like.

### (Embodiment 2)

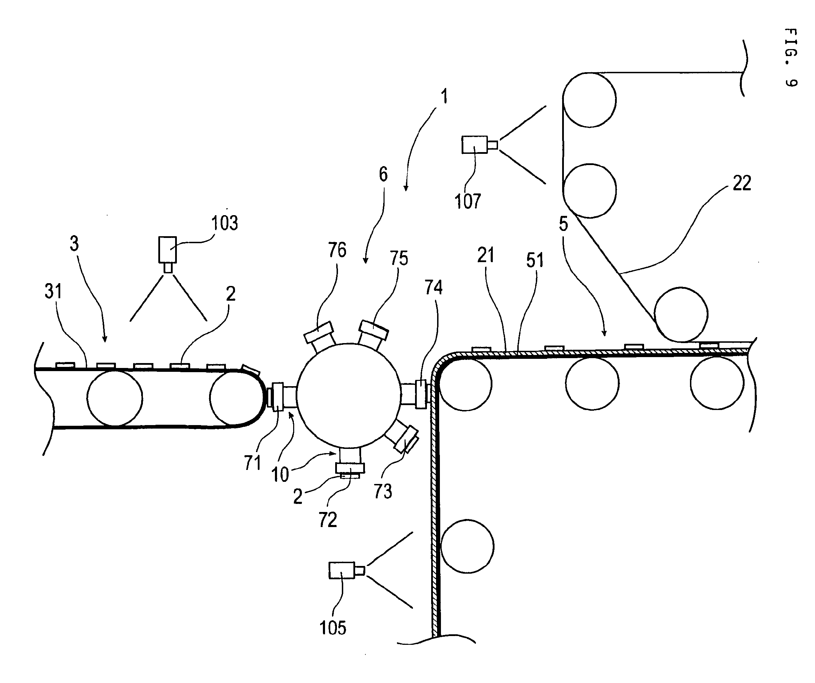

This embodiment is such that the configuration of the second conveying device 5 is changed based on the conveying apparatus in Embodiment 1. This will be described with reference to Figure 9.
A second conveying device 5 in the embodiment is configured to convey a carrier work 21 as a counterpart member on which works 2 are transferred. The transfer device 6 in the embodiment is configured to place the works 2 on predetermined positions on the carrier work 21 conveyed by the second conveying device 5. Further, the conveying apparatus 1 in the embodiment includes a feeding mechanism that feeds a laminate film 22 for laminating a surface of the carrier work 21.

In the embodiment, a continuous material (a continuous sheet material) of nonwoven fabric for substrates of disposable diapers is used as the carrier work 21. Pads of pulp are used as the works 2 to be placed on the carrier work 21. The continuous material of nonwoven fabric on which the pads are placed by the conveying device in the embodiment is cut into pieces of predetermined size in a downstream process.

Plus marks (not shown in figures) indicating target transfer positions are applied on the surface of the carrier work 21 conveyed by the second conveying device 5. An imaging device 105 is used to obtain image data of the carrier work 21 under conveyance, and the image data is processed to recognize positions and a movement speed of the marks. In the conveying apparatus 1 in the embodiment, revolution of end-effectors is controlled based on the positions and the movement speed of the marks. This allows the works 2 to be joined to the surface of the carrier work 21 (on the positions of the markers) with high positional accuracy.

Further, the laminate film 22 is laminated on the surface of the carrier work 21 on which the works 2 are placed. The laminate film 22 in the embodiment is printed with bear characters in order to increase the design effect of the disposable diapers. An unshown control device recognizes the pattern on the laminate film 22 using an imaging device 107 and detects a feeding position thereof. This achieves alignment between the laminate film 22 and the carrier work 21 with high accuracy. An inexpensive optical sensor may be used as the imaging device 107.

Other configurations and the operation and effect are the same as in Embodiment 1. The configuration of the conveying apparatus in the embodiment can be used in various converting machines, printers, labelers, semiconductor producing devices, or the like. For example, the conveying apparatus can be used for a production process of disposable diapers, sanitary napkins, tampons, or face masks.

Further, the conveying apparatus can be used for labeling with seal labels as works 2 and various products such as sanitary products or food products such as snacks as carrier works 21. Further, with a combination of works 2 as various products such as sanitary products or food products such as snacks, and a carrier work 21 and a laminate film 22 as packaging films, the works 2 as the products can be packaged. In this case, edges of the carrier work 21 and the laminate film 22 facing each other can be bonded to provide a bag-like package. Further, the conveying apparatus can be used for a production process of, for example, RF-TAGs. In the production process of the RF-TAGs, an antenna sheet formed with printed antennas is a carrier work 21, and interposers mounted with ICs are works 2.

## Claims

1. A conveying apparatus comprising:
first and second conveying devices configured to hold and convey a work; and a transfer device configured to receive the work from the first conveying device and transfer the work to the second conveying device,
wherein the transfer device comprises two or more end-effectors that revolve along the same circumference to convey the work, and each of the end-effectors is configured to revolve independently of at least any of the other end-effectors.

2. The conveying apparatus according to claim 1, wherein the transfer device includes coaxial rotors that integrally hold one or more end-effectors revolving therewith, and three or more bearings placed coaxially so as to rotatably support at least two coaxial rotors, each of the bearings includes a substantially cylindrical inner ring, a substantially cylindrical outer ring fitted from outside to the inner ring, and a bearing mechanism that allows relative rotation between the inner ring and the outer ring,
the inner ring of one or more middle bearings placed in an axially middle position among the bearings is connected to the outer ring of adjacent another bearing and is configured to integrally rotate therewith, and integrally holds any of the coaxial rotors,
the inner ring of one of bearings placed at axial ends among the bearings is connected to the outer ring of adjacent another bearing and is configured to integrally rotate therewith, and integrally holds any of the coaxial rotors, and the outer ring thereof is secured to a structure member of the transfer device,
the outer ring of the other of the bearings placed at the axial ends among the bearings is connected to the inner ring of adjacent another bearing and is configured to integrally rotate therewith, and the inner ring thereof is secured to a structure member of the transfer device, and
the outer rings integrally connected to the inner rings of the adjacent bearings among the outer rings are connected to an output shaft of an external motor whose rotation is independently controlled.

3. The conveying apparatus according to claim 1, wherein the conveying apparatus comprises control means for controlling a revolution speed and a revolution position in revolution of the each end-effector,
the control means is configured to perform control so that a revolution speed of the end-effector when receiving the work from the first conveying device substantially matches a conveying speed of the first conveying device, and a revolution speed of the end-effector when transferring the work to the second conveying device substantially matches a conveying speed of the second conveying device.

4. The conveying apparatus according to claim 2, further comprising a first measuring portion configured to detect a conveying position and a conveying speed of the work conveyed by the first conveying device.

5. The conveying apparatus according to claim 2, wherein the second conveying device has a conveying surface on which marks are provided, the marks indicating target conveying positions on which the works are transferred by the transfer device, and the transfer device includes a second measuring portion configured to detect the target conveying positions in the second conveying device and a movement speed thereof.

6. The conveying apparatus according to claim 2, wherein a difference between the conveying speed of the first conveying device and the conveying speed of the second conveying device is 80% to 400% of the conveying speed of the first conveying device.

7. The conveying apparatus according to claim 2, wherein the first and the second conveying device each include a roller or a translating conveyor belt, and are configured to convey the work placed on the roller or the conveyer belt.
